# EUROPÄISCHE PATENTANMELDUNG

(11) **EP 2 147 689 A2**
(43) Veröffentlichungstag der Anmeldung: **27.01.2010**
(21) Anmeldenummer: 09163442.8
(22) Anmeldetag: 23.06.2009
(51) Int. Cl.: A61L 31/10, A61L 31/16

(54) **Implantat mit Beschichtung**

(30) Priorität: 21.07.2008 DE 102008040572
(71) Anmelder: Biotronik VI Patent AG, 6341 Baar (CH)
(72) Erfinder: Borck, Alexander, 91086, Aurachtal (DE); Gratz, Matthias, 91054, Erlangen (DE)
(74) Vertreter: Lindner-Vogt, Karin L.

(57) **Zusammenfassung**

Die vorliegende Erfindung betrifft ein Implantat mit einer molekular geprägten Polymerbeschichtung, ein Verfahren zu dessen Herstellung, ein molekular geprägtes Polymer sowie ein Verfahren zu dessen Herstellung und dessen Verwendungen als auch ein Verfahren zur Behandlung oder Prophylaxe von bestimmten Krankheitszuständen durch Implantation eines erfindungsgemäßen Implantats gemäß den jeweiligen unabhängigen Ansprüchen.

## Beschreibung

### Gebiet der Erfindung:

Die vorliegende Erfindung betrifft ein Implantat mit einer Polymerbeschichtung, ein Verfahren zu dessen Herstellung, ein Polymer sowie ein Verfahren zu dessen Herstellung und dessen Verwendungen als auch ein Verfahren zur Behandlung oder Prophylaxe von bestimmten Krankheitszuständen durch Implantation eines erfindungsgemäßen Implantats gemäß den jeweiligen unabhängigen Ansprüchen.

### Stand der Technik:

Implantate sind Stoffe oder Teile, die zur Erfüllung bestimmter Ersatzfunktionen für einen begrenzten Zeitraum oder auf Lebenszeit in den menschlichen oder tierischen Körper eingebracht werden. Im Gegensatz zu Transplantaten bestehen Implantate aus künstlichem Material (Alloplastik). Es wird häufig nach medizinischen, plastischen oder funktionellen Implantaten unterschieden.

Medizinische Implantate haben die Aufgabe, Körperfunktionen zu unterstützen oder zu ersetzen. Je nach Funktion werden sie auch als implantierbare Prothese bezeichnet. Bekannte Vertreter sind z. B. Herzschrittmacher, Hirnschrittmacher bei Parkinson, Herzimplantate, Cochleaimplantate, Implantate in der Zahnmedizin, Stents und Implantate, die dazu dienen, ein Depot eines Arzneistoffes zu bilden sowie verschiedene Formen des Gelenkersatzes.

Plastische Implantate werden in der plastischen Chirurgie, z. B. zum Ersatz von zerstörten Körperteilen oder auch zur Veränderung von bestehenden Körperteilen verwendet.

Funktionelle Implantate dienen zur Überwachung von menschlichen oder tierischen Funktionen, indem z. B. RFID (radio frequency identicifaction)-Chips unter die Haut verpflanzt werden.

Anhand der Vielzahl der vorhandenen Implantattypen kann man erkennen, dass Implantate und deren Anwendung in der Medizin einen großen Stellenwert erlangt haben.

Da bei klassischen Behandlungsprinzipien, wie beispielsweise bei der systemischen Applikation von ein oder mehreren Wirkstoffen, zum Teil erhebliche Nebenwirkungen, wie beispielsweise bei der Tumorbehandlung, zu erwarten sind, gewinnt eine lokale, kontrollierte Wirkstofffreisetzung am Zielort oder in der Nähe davon zunehmende Bedeutung (Local-Drug-Delivery-Konzept; LDD-Konzept). Um diese lokale Applikation von Wirkstoffen durchführen zu können, werden insbesondere Implantatgrundkörper mit ein oder mehreren Wirkstoffen beschichtet, die entweder am Zielort oder in dessen Nähe in einen menschlichen oder tierischen Organismus implantiert werden und so lokal Wirkstoffe freisetzen. Diese klinisch etablierte Methode wird jährlich weltweit millionenfach angewendet und es ist zu erwarten, dass bei Berücksichtigung der demoskopischen Verschiebung innerhalb der Alterspyramide der Bedarf an neuen Werkstoffen sowie neuen Darreichungsformen wächst.

Im orthopädischen Bereich sind im Zusammenhang mit endoprothetischen Implantaten implantat-assozüerte Infektionen und thromboembolische Komplikationen bekannt. Thromboembolie ist ein akuter venöser oder arterieller Gefäßverschluss, der durch einen verschleppten Thrombus, der durch das Anhaften von Thrombozyten an die Oberfläche des Implantates entstehen kann, hervorgerufen wird.

Im Bereich der Herz-Kreislauf-Erkrankungen bieten minimalinvasive Therapieformen zur Aufweitung und Stabilisierung stenosierter Koronargefäße durch die Perkutane Transluminale Coronarangioplastie (PTCA) und die Stentimplantation eine sich immer weiter etablierende Behandlungsmethode. Als Spätkomplikation ist hier neben der Wiederverengung des Gefäßes nach PTCA (sogenannte In-Stent-Restenose, (ISR)) und der Gewebeinflammation vor allem das Risiko einer Thromboembolie zu nennen. Vor diesem Hintergrund werden insbesondere Stents im Stand der Technik bereitgestellt, die mit Wirkstoffen beschichtet sind, die ein oder mehreren Spätkomplikationen entgegenwirken sollen.

Die wirkstofffreisetzenden Implantate des Standes der Technik sind demnach mit ein oder mehreren Wirkstoffen in einer vorgegebenen Konzentration beschichtet und setzen diese gegebenenfalls nach einem definierten Freisetzungsregime in physiologischer Umgebung eines menschlichen oder tierischen Körper frei. Diese wirkstoffbeschichteten Implantate des Standes der Technik ermöglichen jedoch keine flexible auf eine Situation (Auftreten einer Spätkomplikation) bedarfsgerecht abgestimmte Freisetzung von Wirkstoffen bzw. Hemmung von physiologischen Reaktionen als Antwort auf die Implantation des Implantats, beispielsweise inadäquate Proliferation glatter Muskelzellen, überschießende Bildung und Sekretion von Bestandteilen der extrazellulären Matrix, etc.

Die Aufgabe der vorliegenden Erfindung besteht nun darin, ein Implantat bereitzustellen, dass unter physiologischen Bedingungen in einem menschlichen oder tierischen Organismus ein oder mehrere der folgenden Eigenschaften aufweist:
- Bindung an i) ein oder mehrere physiologische Verbindungen eines menschlichen oder tierischen Organismus, insbesondere
   - zur Anreicherung von Wachstumsfaktoren, Enzymen zur Modifikation/Reduktion extrazellulärer Matrix, Anreicherung von Enzymen zur Fibrinlösung der Anreicherung von Enzymen zur Aktivierung von systemisch verabreichten Prodrugs,
   - Hemmung von Rezeptoren für Wachstumsfaktoren oder Hemmung von Rezeptoren der Zellaktivierung und/oder
- Bindung ii) ein oder mehrerer systemisch verabreichter Wirkstoffe.

### Zusammenfassung der Erfindung:

Die vorliegende Aufgabe wird teilweise oder vollständig durch die Gegenstände der unabhängigen Ansprüche gelöst.

Demnach betrifft ein erster Erfindungsgegenstand ein Implantat zur Implantation in einen menschlichen oder tierischen Organismus mit einer Polymerbeschichtung, dadurch gekennzeichnet, dass die Polymerbeschichtung molekular geprägt ist und die molekulare Prägung geeignet ist unter physiologischen Bedingungen in einem menschlichen oder tierischen Organismus
i) ein oder mehrere physiologische Verbindungen eines menschlichen oder tierischen Organismus und/oder
ii) ein oder mehrere systemisch verabreichte Wirkstoffe zu binden.

Ein zweiter Erfindungsgegenstand betrifft ein Verfahren zur Herstellung eines erfindungsgemäß polymerbeschichteten Implantats.

Ein dritter Erfindungsgegenstand betrifft ein molekular geprägtes Polymer geeignet als Beschichtung für ein erfindungsgemäßes Implantat, **dadurch gekennzeichnet, dass** die molekulare Prägung geeignet ist unter physiologischen Bedingungen in einem menschlichen oder tierischen Organismus
i) ein oder mehrere physiologische Verbindungen eines menschlichen oder tierischen Organismus und/oder
ii) ein oder mehrere systemisch verabreichbare Wirkstoffe zu binden.

Ein vierter Erfindungsgegenstand betrifft ein Verfahren zu Herstellung eines erfindungsgemäßen molekular geprägten Polymers, **dadurch gekennzeichnet, dass** das Verfahren folgende Schritte umfasst:
a) Bereitstellung von
   i) ein oder mehreren physiologischen Verbindungen eines menschlichen oder tierischen Organismus und/oder
   ii) ein oder mehreren systemisch verabreichbaren Wirkstoffen,
b) Bereitstellung von ein oder mehreren polymerisierbare Materialien,
c) Bereitstellung eines oder mehrerer Polymerisationsinitiatoren,
d) Vermischen i) der Verbindung oder der Verbindungen oder ii) des oder der Wirkstoffe aus Schritt a) mit den polymerisierbaren Materialien aus Schritt b) sowie dem oder den Polymerisationsinitiatoren aus Schritt c),
e) Polymerisieren der Mischung aus Schritt d) und
f) Entfernen i) des oder der Verbindungen und/oder ii) des oder der Wirkstoffe zumindest aus der Oberfläche des Polymers durch Waschung des Polymers aus Schritt e) mit einem oder mehreren geeigneten Lösungsmittel und/oder Lösungsmittelgemisch.

Ein fünfter Gegenstand der vorliegenden Erfindung betrifft eine Verwendung eines erfindungsgemäßen molekular geprägten Polymers als Beschichtung für ein erfindungsgemäßes Implantat.

Ein sechster Erfindungsgegenstand betrifft eine Verwendung eines erfindungsgemäßen molekular geprägten Polymers zur Herstellung eines erfindungsgemäßen Implantats.

Ein siebter Erfindungsgegenstand betrifft ein Verfahren zur Behandlung oder Prophylaxe einer Stenose und die Behandlung von Erkrankungen der Gefäße oder durch diese Gefäße versorgter Gewebe, einschließlich Tumoren, **dadurch gekennzeichnet, dass** ein erfindungsgemäßes Implantat in einen menschlichen oder tierischen Organismus implantiert wird.

Bevorzugte Ausgestaltungen der erfindungsgemäßen Gegenstände werden in den abhängigen Ansprüchen sowie der nachfolgenden detaillierten Beschreibung dargestellt und sind, sofern sinnvoll, untereinander kombinierbar.

### Kurze Figurenbeschreibung:

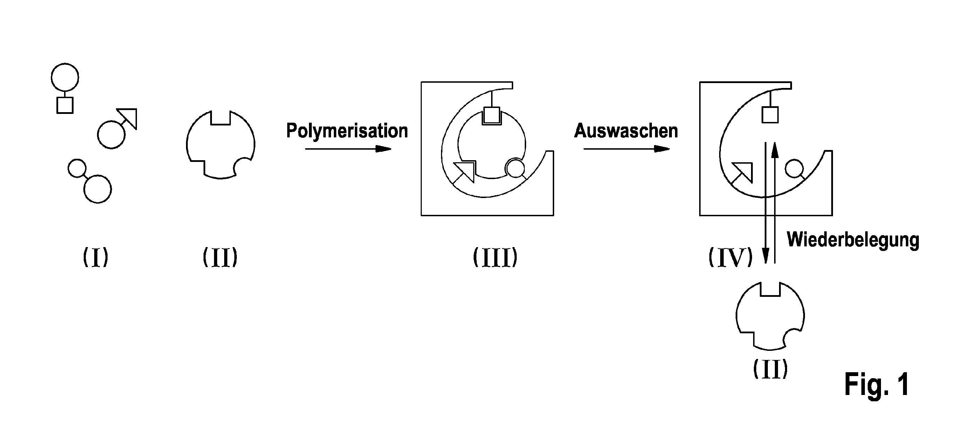

Fig. 1 zeigt ein schematisiertes Verfahren zur erfindungsgemäßen Herstellung eines molekular geprägten Polymers.

### Detaillierte Beschreibung der Erfindung:

Die Erfindung beruht auf der überraschenden Erkenntnis, dass durch ein erfindungsgemäßes Implantat mit einer molekular geprägten Polymerbeschichtung bzw. ein erfindungsgemäß molekular geprägtes Polymer nach den jeweiligen unabhängigen Ansprüchen i) ein oder mehrere physiologische Verbindungen eines menschlichen oder tierischen Organismus und/oder ii) ein oder mehrere Wirkstoffe unter physiologischen Bedingungen in einem menschlichen oder tierischen Organismus an die Oberfläche des Polymers gebunden werden und somit unter Ausnutzung körpereigener Mechanismen
- Wachstumsfaktoren zur Verbesserung der Endothelialisierung, insbesondere im Gefäßsystem bei Stenosen, Enzyme zur Modifikation/Reduktion extrazellulärer Matrix, Enzyme zur Fibrinlösung und/oder Enzyme zur Spaltung von systemisch verabreichten Prodrugs angereichert werden,
- Rezeptoren für Wachstumsfaktoren zur Proliferationskontrolle oder Rezeptoren der Zellaktivierung zur Manipulation von Immunzellen gehemmt werden und/oder
- Systemisch verabreichte Wirkstoffe an die Polymeroberfläche gebunden werden, was
   - eine nachträglich und damit bedarfsgerechte Wirkstoffbeladung des Implantats und damit auch Wirkstofffreisetzung (beispielsweise notwendig bei Vorliegen weiterer Krankheitsbilder, insbesondere Diabetes mellitus) ermöglicht,
   - den Erhalt einer Wirkstoffkonzentration über einen vordefinierten Zeitraum und damit Wiederholungsapplikationen ermöglicht,
   - einen Wechsel von Wirkstoffen von beispielsweise von betont proliferationshemmend (z.B. Paclitaxel) zu betont endothelfördernd oder antithrombogen wirkend ermöglicht,
   - eine parallele lokale Vasodilationstherapie ermöglicht,
   - ein Einwirken auf Effekte einer lokalen pH-Wert-Verschiebung insbesondere beim Abbau von degradierbaren Metallstents, vorzugsweise Magnesiumstents ermöglicht.

Implantate bzw. Implantatgrundkörper im Sinne der vorliegenden Erfindung können alle medizinischen, plastischen und/oder funktionellen Implantate bzw. Implantatgrundkörper darstellen und werden beispielsweise ausgewählt aus der Gruppe bestehend aus Herzschrittmacher; Hirnschrittmacher; Herzimplantate; Schrittmacherelektroden; Defibrillationselektroden; Cochleaimplantate; Implantate für Zahnmedizin; Endoprothesen, bevorzugt für Kniegelenke und/Hüftgelenke; Depotimplantate, die dazu dienen, ein Depot eines Wirkstoffs zu bilden; degradierbare oder dauerhafte, koronare oder periphere Stents; degradierbare oder dauerhafte Stents für andere Hohlräume, vorzugsweise die Speiseröhre, den Gallengang, die Harnröhre, die Prostata oder die Luftröhre; und local drug delivery (LDD)-Implantate, die vorzugsweise endovaskulär im Blut oder anderen Hohlräumen implantiert werden.

In einer bevorzugten Ausgestaltung der vorliegenden Erfindung werden erfindungsgemäße Implantate ausgewählt aus der Gruppe bestehend aus Herzschrittmacher; Herzimplantate; Schrittmacherelektroden; Defibrillationselektroden; degradierbare oder dauerhafte, koronare oder periphere Stents; und local drug delivery (LDD)-Implantate, die vorzugsweise endovaskulär im Blut oder anderen Hohlräumen implantiert werden.

In einer besonders bevorzugten Ausgestaltung der vorliegenden Erfindung werden erfindungsgemäße Implantate ausgewählt aus der Gruppe der dauerhaften oder degradierbaren koronaren Stents (Koronarstents), wobei das Stentgrundkörpermaterial Metall und/oder Polymer umfassen kann.

Die ursprünglichen mechanischen Funktionen eines Koronarstents, beispielsweise die Dilatierbarkeit, der geringe Recoil, die Stabilität über eine gewünschte Zeitdauer (im Falle degradierbarer Stents, beispielsweise bestehend aus Magnesium und seinen Legierungen) sowie die Flexibilität sollen in erfindungsgemäßen Stents als erfindungsgemäße Implantate bevorzugt vorhanden sein.

Im Folgenden werden erfindungsgemäß zu verwendende Implantat-Materialien, bevorzugt Stentgrundkörper-Materialien sowie bevorzugte Ausgestaltungen hiervon beschrieben:

### Degradierbarer Implantatgrundkörper, vorzugsweise degradierbarer Stentgrundkörper:

Im Sinne der vorliegenden Erfindung bedeutet "degradierbarer Implantat(grundkörper)", bevorzugt "degradierbarer Stent(grundkörper)", dass der Grundkörper in physiologischer Umgebung, insbesondere im Gefäßsystem eines menschlichen oder tierischen Organismus degradiert, d. h., so abgebaut wird, dass der Stent seine Integrität verliert. Vorzugsweise degradieren erfindungsgemäß degradierbare Grundkörper erst dann, wenn die Funktionen des Implantats nicht mehr physiologisch sinnvoll bzw. notwendig sind. Bei degradierbaren Stents bedeutet das, dass der Stent vorzugsweise erst dann degradiert oder seine Integrität verliert, wenn das traumatisierte Gewebe des Gefäßes verheilt ist und der Stent somit nicht länger im Gefäßlumen verbleiben muss.

### Metallischer Grundkörper:

In einer bevorzugten erfindungsgemäßen Ausgestaltung umfasst oder besteht der degradierbare Werkstoff bevorzugt aus einem metallischen Werkstoff, der eine biokorrodierbare Legierung darstellt, wobei die Hauptkomponente der Legierung ausgewählt wird aus der Gruppe Magnesium, Eisen, Zink und/oder Wolfram; insbesondere wird für einen degradierbaren metallischen Werkstoff eine Magnesiumlegierung bevorzugt.

Die Legierung, insbesondere umfassend Magnesium, Eisen, Zink und/oder Wolfram, ist so in ihrer Zusammensetzung zu wählen, dass sie biokorrodierbar ist. Als biokorrodierbar im Sinne der vorliegenden Erfindung werden Legierungen bezeichnet, bei denen in physiologischer Umgebung ein Abbau (Degradierung) stattfindet, der letztendlich dazu führt, dass der gesamte Stent oder der aus dem Werkstoff gebildete Teil des Stents seine mechanische Integrität verliert. Unter Legierung wird vorliegend ein metallisches Gefüge verstanden, dessen Hauptkomponente Magnesium, Eisen, Zink und/oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, weiter bevorzugt mehr als 70 Gew.%. Eine Magnesium-Legierung ist hiervon besonders bevorzugt.

Ist der erfindungsgemäße Werkstoff eine Magnesium-Legierung, so enthält diese vorzugsweise Yttrium und weitere Seltenerdmetalle, da sich eine derartige Legierung aufgrund ihrer physiko-chemischen Eigenschaften und ihrer hohen Biokompatibilität, insbesondere auch ihrer Abbauprodukte, auszeichnet.

Besonders bevorzugt werde die in EP 1 419 793 B1 beschriebenen Yttrium (W) und Seltene Erden (E) enthaltenden biokorrodierbaren Magnesiumlegierungen (WE43 & WE54 von Magnesium Elektron) mit einem Anteil von Magnesium >90 Gew., Yttrium 3,7 - 5,5 Gew.%, Seltenerdmetallen 1,5 - 4,4 Gew.% und Rest < 1 Gew.%, zur Herstellung von Implantaten (Stents) eingesetzt.
Diese Magnesiumlegierungen bestätigen bereits experimentell und in ersten klinischen Versuchen ihre besondere Eignung, d. h. sie zeigen eine hohe Biokompatibilität, günstige Verarbeitungseigenschaften, gute mechanische Kennwerte und ein für die Einsatzzwecke adäquates Korrosionsverhalten.
Unter der Sammelbezeichnung "Seltene Erden" SE werden in der vorliegenden Anmeldung Scandium (21) und Yttrium (39) sowie die "Leichten Seltenen Erden" LSE Lanthan (57), Cer (58), Neodym (60), und Promethium (61) und die "Schweren Seltenen Erden" SSE Samarium (62), Europium (63), Gadolinium (64), Terbium (65), Dysprosium (66), Holmium (67), Erbium (68), Thulium (69), Ytterbium (70) und Lutetium (71) verstanden.

### Polymer-Grundkörper:

Erfindungsgemäße Implantatgrundkörper, bevorzugt Stentgrundkörper, können gemäß einer weiteren alternativen erfindungsgemäßen Ausgestaltung degradierbares Polymer umfassen oder daraus bestehen, vorzugsweise ausgewählt aus der Gruppe bestehend aus Polydioxanon; Polyglycolid; Polycaprolacton; Polyhydroxyvaleriansäure; Polyhydroxybuttersäure; Polylactide, vorzugsweise Poly(L-lactid), Poly(D-lactid), Poly(D,L-lactid) und Blends sowie Copoylmere, und vorzugsweise Poly(L-lactid-co-glycolid), Poly(D,L-lactid-co-glycolid), Poly(L-lactid-co-D-L-lactid), Poly(L-lactid-co-trimethylencarbonat) und Tri-blockcopolymere; Polysaccharide, vorzugsweise Chitosan, Levan, Hyaluronsäure, Heparin, Dextran und Cellulose.

### Dauerhafter Implantatgrundkörper, vorzugsweise dauerhafter Stentgrundkörper:

Im Gegensatz zum degradierbaren Grundkörper wird der "dauerhafte Implantatgrundkörper", vorzugsweise der "dauerhafte Stentgrundkörper" in physiologischer Umgebung im menschlichen oder tierischen Organismus im Wesentlichen nicht abgebaut, so dass er seine Integrität nicht verliert.

In einer weiteren erfindungsgemäßen bevorzugten Ausgestaltung umfasst oder besteht der Grundkörper eines dauerhaften Implantats, vorzugsweise eines dauerhaften Stents, aus einem Formgedächtnis-Material, vorzugsweise aus einem oder mehreren Materialien ausgewählt aus der Gruppe der Nickel-Titan-Legierungen und Kupfer-Zink-AluminiumLegierungen, weiter bevorzugt Nitinol.

In einer besonders bevorzugten erfindungsgemäßen Ausgestaltung umfasst oder besteht der Grundkörper eines dauerhaften Implantats, vorzugsweise eines dauerhaften Stents aus Edelstahl, vorzugsweise aus Cr-Ni-Fe-Stahl, hier bevorzugt die Legierung 316L, oder einem Co-Cr-Stahl.

In einer weiteren bevorzugten Ausgestaltung kann der Implantatgrundkörper, vorzugsweise Stentgrundkörper zusätzlich Kunststoff, vorzugsweise Polyetherurethan, und/oder Keramik und/oder weitere Polymerbeschichtungen umfassen.

Werden im Sinne der vorliegenden Erfindung endovaskulär implantierbare Stents, vorzugsweise Koronarstents als implantierbare Grundkörper verwendet, so können alle üblichen Stentgeometrien für die vorliegende Erfindung verwendet werden. Insbesondere bevorzugt sind Stentgeometrien, die insbesondere in US 6,896,695, US 2006/241742, US 5,968,083 (Tenax), EP 1 430 854 (Helix-Design), US 6,197, 047 und EP 0 884 985 beschrieben werden.

Der erfindungsgemäße Implantatgrundkörper, vorzugsweise Stentgrundkörper einschließlich der vorstehenden bevorzugten Ausgestaltungen weist eine Polymerbeschichtung auf, die erfindungsgemäß molekular geprägt ist. Das Polymer für die erfindungsgemäße Polymerbeschichtung wird vorzugsweise ausgewählt aus der Gruppe bestehend aus Copolymerisaten von ein oder mehreren Acrylaten und/oder Metacrylaten mit Acrylacetat (weist hydrophile Gruppen auf, die gut zu OH-Gruppen verseift werden können), Acrylamin, Acrylsäuren, Vinylpyrrolidon, Styrol (Wechselwirkung mit aromatischen Epitopen), Vinylpyridin oder Vinylsaccharid-Copolmerisaten ausgewählt. Vinylsaccharide lassen sich gemäß der folgenden Literatur herstellen: http://www.digibib.tu-bs.de/?docid=0000124; ISBN 3-89825-567-0, Anke Wiegand und http://bib1lp1.rz.tubs.de/docportal/content/below/index.xml. Die Auswahl der Monomere richtet sich stark nach dem Templat, auf das das Polymer mit Bindungsaffinität versorgt werden soll. Soll ein Polymer mit Selektivität auf eine aromatische Verbindung bzw. Naphthalin erzeugt werden, ist die Verwendung von Styrol sinnvoll. Bei amingruppentragenden Molekülen eignen sich Monomere, die eine Säuregruppe oder OH-Gruppe tragen. Die beschriebenen Vinylsaccharide können mit einer großen Anzahl von Templaten Weckelwirkungen eingehen.

Gemäß einer bevorzugten erfindungsgemäßen Ausgestaltung ist die molekulare Prägung des erfindungsgemäßen Implantats, vorzugsweise Stents geeignet unter physiologischen Bedingungen in einem menschlichen oder tierischen Organismus i) ein oder mehrere physiologische Verbindungen zu binden, der oder die ausgewählt werden aus der Gruppe bestehend aus Wachstumsfaktoren, bevorzugt Transforming Growth Factor, Fibroblast Growth Factor, Angiopoietin 2 und/oder Vascular Endothelial Growth Factor, Enzymen zur Modifikation oder Reduktion extrazellulärer Matrix, bevorzugt Matrix-Metalloproteinasen und dabei besonders bevorzugt Matrix-Metalloproteinase 1, Enzymen zur Fibrinlösung wie, aber nicht ausschließlich, dem Plasmin, bevorzugt aber seinen Aktivatoren oder in Kombination mit diesen, dabei bevorzugt Gewebsplasminogenaktivator und/oder Urokinase Plasminogenaktivator, Enzymen zur Aktivierung von systemisch verabreichten Prodrugs, zum Beispiel Esterasen, Enzyme aus der Reihe der Hydrolasen insbesondere der Lipasen und Proteasen, Enzyme aus der Reihe der Isomerasen, Rezeptoren für Wachstumsfaktoren, bevorzugt aber nicht beschränkt auf Rezeptoren für den Platelet Derived Growth Factor.

Im Sinne der vorliegenden Erfindung bedeutet "Wirkstoff" eine Substanz oder eine Verbindung, die in einem menschlichen oder tierischen Organismus nicht physiologisch vorhanden ist und im menschlichen oder tierischen Organismus eine biologische Reaktion hervorruft.

In einer bevorzugten Ausgestaltung des erfindungsgemäßen Implantats, vorzugsweise des erfindungsgemäßen Stents wird die Polymer-Beschichtung vorzugsweise dahingehend molekular geprägt, dass sie an ii) ein oder mehrere systemisch verabreichte Wirkstoffe bindet, welche eine Kontrolle der Bildung extrazellulärer Matrix bzw. Intima-Proliferation ermöglichen, d. h. proliferationshemmend wirken, beispielsweise Paclitaxel oder Rapamycin und seine Derivate, endothelfördernd wirken, beispielsweise Statine, wie zum Beispiel Atorvastatin oder Cerivastatin, antithrombogen und/oder antikoagulierend wirken, beispielsweise Dipyridamol, Heparin, Cumarinderivate, beispielsweise Marcumar, Acetylsalicylsäure und/oder Clopidogrel, vasodilatierend wirken, beispielsweise Dipyridamol, NO oder Stickstoffmonoxiddonatoren wie zum Beispiel Isosorbiddinitrat, Nitroprussid oder Molsidomin, Calciumantagonisten, ACE-Hemmer wie Captopril, Angiotensin-II-Rezeptor-Subtyp-1-Antagonisten wie zum Beispiel Losartan, Kaliumkanalöffner wie Minoxidil oder Diazoxid und/oder Endothelin-Rezeptorantagonisten wie zum Beispiel Bosentan oder Sitaxentan, und/oder dem biologischen Effekt einer lokalen pH-Wert-Verschiebung in den basischen Bereich bei degradierbaren Metallstents, vorzugsweise Magnesium-Stents entgegenwirken kann. Biologische Effekte, die bei einer lokalen pH-Wert-Verschiebung in den basischen Bereich nach der Implantation von Metallstents, vorzugsweise Magnesium-Stents auftreten, sowie Wirkstoffe, die diesen biologischen Effekten entgegenwirken, werden in DE 10 2006 038 235 A1 beschrieben und werden in soweit durch Referenz auf DE 10 2006 038 235 A1 hierein inkorporiert.

In einer weiteren bevorzugten Ausgestaltung umfasst die Polymerbeschichtung des erfindungsgemäßen Implantats, vorzugsweise des erfindungsgemäßen Stents, zusätzlich ein oder mehrere Wirkstoffe, vorzugsweise ausgewählt aus der Gruppe bestehend aus Wirkstoffen welche eine Kontrolle der Bildung extrazellulärer Matrix bzw. Intima-Proliferation ermöglichen, d. h. proliferationshemmend wirken, beispielsweise Paclitaxel oder Rapamycin und seine Derivate, endothelfördernd wirken, beispielsweise Statine, wie zum Beispiel Atorvastatin oder Cerivastatin, antithrombogen und/oder antikoagulierend wirken, beispielsweise Dipyridamol, Heparin, Cumarinderivate, beispielsweise Marcumar, Acetylsalicylsäure und/oder Clopidogrel, vasodilatierend wirken, beispielsweise Dipyridamol, NO oder Stickstoffmonoxiddonatoren wie zum Beispiel Isosorbiddinitrat, Nitroprussid oder Molsidomin, ACE-Hemmer wie Captopril, Angiotensin-II-Rezeptor-Subtyp-1-Antagonisten wie zum Beispiel Losartan, Kaliumkanalöffner wie Minoxidil oder Diazoxid und/oder Endothelin-Rezeptorantagonisten wie zum Beispiel Bosentan oder Sitaxentan, und/oder dem biologischen Effekt einer lokalen pH-Wert-Verschiebung in den basischen Bereich bei degradierbaren Metallstents, vorzugsweise Magnesium-Stents, entgegenwirken können. Biologische Effekte, die bei einer lokalen pH-Wert-Verschiebung in den basischen Bereich nach der Implantation von Metallstents, vorzugsweise Magnesium-Stents auftreten, sowie Wirkstoffe, die diesen biologischen Effekten entgegenwirken, werden in DE 10 2006 038 235 A1 beschrieben und werden in soweit durch Referenz auf DE 10 2006 038 235 A1 hierein inkorporiert.

### Ausführungsbeispiel

### Herstellung einer Polymermatrix mit Bindungsselektivität zu Dipyridamol

Zusammensetzung der Polymere: 60 % Methacrylsäure, 30 % Acrylsäure oder Acrylat oder Acrylat-Derivat , 10 % Vinylpyrrolidon in Aceton alternativ eignen sich THF und Ethanol, wobei in diesen Lösungsmitteln die Wirkstoffkonzentration limitiert ist.

Nach dem Lösen von 10g Wirkstoff (0,5 -10 g) zu 100 ml Aceton werden 6 g Methacrylsäure, 3 g Acrylsäure und 1 g Vinylpyrrolidon zugegeben.

Die Mischung wird gerührt und mit 2 mg AIBN versetz und unter einer UV-Lampe (230 nm) polymerisiert. Über die erste Stunde der Polymerisierung wird die Reaktion alle 15 Minuten mit einem Vortex (10 Sek.) stark geschüttelt.

Der entstandene Feststoff wird nach 6 Stunden entnommen, zerstoßen und mit 100 ml Wasser gewaschen.

Die Freisetzung von Dipyridamol aus der Polyermatrix kann mittels HPLC-Analytik bestimmt werden.

### Beschichtung eines Stentgrundkörpers mit molekular geprägtem und beladenem Polymer

Optional wird der Stentgrundkörper unter Feuchtigkeitsausschluss mit Methyl-2-cyanoacrylat besprüht. Die Beschichtung mit Methyl-2-cyanoacrylat eignet sich insbesondere als Haftvermittlerschicht für das anschließend aufgebrachte molekular geprägte Polymer. Der feuchte Stent wird daraufhin in eine Staubkammer überführt, in der sich das pulverisierte und gewaschene Polymer mit molekularer Erkennung auf das Templat (Dipyridamol) befindet. Nach der Abscheidung des molekular geprägten Polymers als Staub auf dem Stent, wird der Stent aus der Staubkammer entnommen und einer Luftfeuchtigkeit von min. 60 % ausgesetzt.

Die Reaktion ist im Folgenden gezeigt:

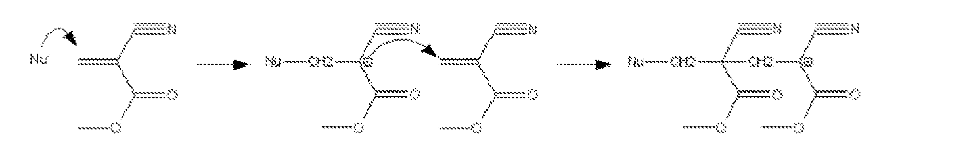

Wasser wirkt hier als Nucleophil und startet die anionische Polymerisation. Der Polymerstab wird so an die Implantatoberfläche geklebt.

Weitere bevorzugte Verbindungen, die als Haftvermittlerschicht auf die Stentoberfläche aufgebracht werden können, sind ausgewählt aus der Gruppe bestehend aus Derivaten von Cyanoacrylat und von Diisocyanat. Besonders bevorzugte Derivate von Diisocyanat sind Hexamethylendiisocyanat (HMDI), Toluoldüsocyanat (TDI), Diisocyanatodicyclohexylmethan (H12MDI) und Isophorondiisocyanat (IPDI). Die Beschichtung der Stentoberfläche mit einem Haftvermittler ist dann besonders vorteilhaft, um erhöhte Mengen des pulverisierten, molekular geprägten Polymers auf der Stentoberfläche aufzubringen.

Bevorzugte Ausgestaltungen zu den Polymermaterialien bzw. i) den ein oder mehreren physiologischen Verbindungen eines menschlichen oder tierischen Organismus und/oder ii) den ein oder mehreren systemisch verabreichten Wirkstoffen aus dem ersten Erfindungsgegenstand können ebenfalls bevorzugte Ausgestaltungen für den dritten erfindungsgemäßen Gegenstand, nämlich das erfindungsgemäße molekular geprägte Polymer selbst sein.

Gemäß dem vierten Erfindungsgegenstand werden nach Schritt a) i) ein oder mehrere physiologische Verbindungen eines menschlichen oder tierischen Organismus und/oder ii) ein oder mehrere systemisch verabreichbare Wirkstoffe bereitgestellt. Bevorzugte Ausgestaltungen hierzu, die bereits zu dem ersten Erfindungsgegenstand beschrieben wurden, können ebenfalls auf den vierten Erfindungsgegenstand angewendet werden.

Gemäß Schritt b) des erfindungsgemäßen Herstellverfahrens für das molekular geprägte Polymer werden ein oder mehrere polymerisierbare Materialien bereitgestellt. Bevorzugte Ausgestaltungen hiervon wurden ebenfalls bereits zu dem ersten erfindungsgemäßen Gegenstand beschrieben und die Monomere hierzu sind ebenfalls auf den vierten Erfindungsgegenstand anwendbar.

Gemäß Schritt c) des erfindungsgemäßen Herstellverfahrens eines molekular geprägten Polymers werden ein oder mehrere Polymerisationsinitiatoren bereitgestellt. Polymerisationsinitiatoren im Sinne der vorliegenden Erfindung sind üblicherweise alle Substanzen oder Verbindungen, welche eine Polymerisation der in Schritt b) des erfindungsgemäßen Verfahrens bereitgestellten polymerisierbaren Materialien ermöglichen. Bevorzugt werden der oder die Polymerisationsinitiatoren ausgewählt aus der Gruppe bestehend aus 2,2-Aco-bis-Isobutyronitril (AIBN).

Gemäß Schritt d) des erfindungsgemäßen Herstellverfahrens eines molekular geprägten Polymers werden i) das oder die Verbindungen oder ii) das oder die Wirkstoffe aus Schritt a) mit den polymerisierbaren Materialien aus Schritt b) sowie dem oder den Polymerisationsinitiatoren aus Schritt c) vermischt. Dies kann zum einen in Substanz aber auch in einem geeigneten Lösungsmittel bzw. Lösungsmittelgemisch durchgeführt werden. In einer bevorzugten Ausgestaltung werden das oder die Verbindungen aus Schritt a) in Schritt d) zuerst in einem Lösungsmittel oder Lösungsmittelgemisch gelöst. Hierfür können alle üblicherweise geeigneten Lösungsmittel bzw. Lösungsmittelgemische, die ebenfalls mischbar mit den polymerisierbaren Materialien aus Schritt b) sind, ausgewählt werden. Bevorzugt wird das Lösungsmittel oder Lösungsmittelgemisch ausgewählt aus der Gruppe bestehend aus Aceton, Dioxan, Chloroform, Tetrahydrofuran und/oder Wasser.

Aceton ist als Lösungsmittel besonders bevorzugt, da im Gegensatz zu Ethanol und Tetrahydrofuran keine, bzw. lediglich eine geringe Beschränkung im Hinblick auf die Konzentration der zu lösenden Verbindungen aus Schritt a) zu erwarten ist.

Gemäß Schritt e) des erfindungsgemäßen Herstellverfahrens für ein molekular geprägtes Polymer wird die Mischung aus Schritt d) gemäß üblicher Verfahren polymerisiert. In einer bevorzugten Ausgestaltung wird die Polymerisation in Schritt e) mittels UV-Strahlung hervorgerufen.

Gemäß Schritt f) des erfindungsgemäßen Herstellverfahrens für ein molekular geprägtes Polymer werden i) der oder die Verbindungen oder ii) der oder die Wirkstoffe zumindest aus der Oberfläche des Polymers durch Waschung des Polymers aus Schritt e) mit einem oder mehreren geeigneten Lösungsmitteln oder Lösungsmittelgemischen entfernt, so dass das molekular geprägte Polymer unter physiologischen Bedingungen geeignet ist, i) das oder die physiologischen Verbindungen und/oder ii) das oder die systemisch verabreichbaren Wirkstoffe erneut zu binden. Es können hierfür üblicherweise alle geeigneten Lösungsmittel und/oder Lösungsmittelgemische ausgewählt werden, welche zu einer Entfernung i) des oder der Verbindungen oder ii) des oder der Wirkstoffe führen. In einer bevorzugten Ausgestaltung werden das oder die geeigneten Lösungsmittel und/oder Lösungsmittelgemische ausgewählt aus der Gruppe bestehend aus Methanol, Ethanol und/oder Wasser.

Vorzugsweise wird der Waschschritt f) zwei-, drei- oder mehrmals durchgeführt. In einer besonders bevorzugten Ausgestaltung wird zuerst ein Lösungsmittelgemisch aus Methanol:Ethanol in einem Volumenverhältnis im Bereich von 4:1 zu 1:4, weiter bevorzugt von 1:1 verwendet und gegebenenfalls anschließend mit Wasser gewaschen.

Gemäß dem fünften Erfindungsgegenstand wird die Verwendung eines erfindungsgemäßen molekular geprägten Polymers als Beschichtung für ein erfindungsgemäßes Implantat beansprucht. Die bevorzugten Ausgestaltungen für das erfindungsgemäße Implantat gemäß erstem Erfindungsgegenstand und/oder dem molekular geprägten Polymer gemäß drittem Erfindungsgegenstand sowie dem Herstellverfahren für ein molekular geprägtes Polymer gemäß viertem Erfindungsgegenstand sind ebenfalls auf den fünften Erfindungsgegenstand anwendbar.

Der sechste Erfindungsgegenstand betrifft die Verwendung eines erfindungsgemäßen molekular geprägten Polymers zur Herstellung eines erfindungsgemäßen polymerbeschichteten Implantats. Auch hier sind die bevorzugten Ausgestaltungen eines erfindungsgemäßen Implantats gemäß des ersten Erfindungsgegenstands, eines erfindungsgemäßen molekular geprägten Polymers gemäß drittem Erfindungsgegenstand sowie die Herstellverfahren für ein erfindungsgemäßes Implantat bzw. ein molekular geprägtes Polymer gemäß zweitem und viertem Erfindungsgegenstand auf den sechsten Erfindungsgegenstand anwendbar.

Schließlich wird gemäß dem siebten Erfindungsgegenstand ein Verfahren zur Behandlung oder Prophylaxe einer Stenose oder zur Behandlung von Erkrankungen der Gefäße oder durch diese Gefäße versorgter Gewebe, einschließlich Tumoren, beansprucht, dass dadurch gekennzeichnet ist, dass ein erfindungsgemäßes Implantat in einen menschlichen oder tierischen Organismus implantiert wird. Auch hier sind die bevorzugten Ausgestaltungen für das erfindungsgemäße Implantat gemäß erstem Erfindungsgegenstand, gegebenenfalls unter Heranziehung des erfindungsgemäßen Herstellverfahrens für das erfindungsgemäße Implantat nach dem zweiten Erfindungsgegenstand und gegebenenfalls dem erfindungsgemäßen molekular geprägten Polymer gemäß drittem Erfindungsgegenstand sowie seinem Herstellverfahren nach dem vierten Erfindungsgegenstand ebenfalls auf die bevorzugten Ausgestaltungen für den siebten Erfindungsgegenstand anwendbar.

### Figurenbeschreibung:

Fig. 1 zeigt eine schematisierte Darstellung eines erfindungsgemäßen Herstellverfahrens für ein erfindungsgemäß molekular geprägtes Polymer.

In Figur 1 stellt (I) die polymerisierbaren Materialien gemäß Schritt b) des erfindungsgemäßen Herstellverfahrens nach dem vierten Erfindungsgegenstand dar.

(II) stellt i) das oder die physiologischen Verbindungen eines menschlichen oder tierischen Organismus und/oder ii) das oder die systemisch verabreichbaren Wirkstoffe gemäß Schritt a) (das oder die sogenannten Template) des erfindungsgemäßen Herstellverfahrens für ein erfindungsgemäßes molekular geprägtes Polymer nach dem vierten Erfindungsgegenstand dar.

Nach Polymerisation liegen die polymerisierbaren Materialien (I) mit dem oder den Templaten (II) als Verbund (III) vor. Die Verbindungen zwischen (I) und (II) können kovalente, ionische, Wasserstoffbrücken- oder andere Verbindungen darstellen. Üblicherweise werden solche Verbindungen gewählt, die aufgrund der Waschvorgänge reversibel gelöst werden können, um eine Anbindung von i) der oder den physiologischen Verbindungen und/oder ii) dem oder den systemisch verabreichten Wirkstoffen zu ermöglichen.

Demnach umfasst das erfindungsgemäß geprägte Polymer (IV) ein oder mehrere vorzugsweise selektive Bindungsstellen für das oder die Template (II), also i) das oder die physiologischen Verbindungen eine menschlichen oder tierischen Organismus und/oder ii) das oder die systemisch verabreichten Wirkstoffe, die unter physiologischen Verbindungen dann wieder mit dem erfindungsgemäß geprägten Polymer eine Bindung eingehen.

Die bevorzugten Ausgestaltungen des dritten bzw. vierten Erfindungsgegenstandes sind ebenfalls auf die Figurenbeschreibung anwendbar.

### Ausführungsbeispiele

Die vorliegende Erfindung wird im Folgenden durch Ausführungsbeispiele beschrieben, die jedoch den Schutzumfang der erfindungsgemäß beanspruchten Gegenstände gemäß unabhängiger Ansprüche nicht limitieren.

### Ausführungsbeispiel 1: Herstellung eines erfindungsgemäß molekular auf Dipyridamol geprägten Polymers

Es werden 10 g Dipyridamol-Wirkstoff, ca. 100 ml Aceton, 6 g Methacrylsäure, 3 g Acrylsäure und 1 g Vinylpyrrolidon und 2 mg 2,2-Azobisisobutyronitril (AIBN) bereitgestellt (Schritte a) bis c) des erfindungsgemäßen Herstellverfahrens für das erfindungsgemäße molekular geprägte Polymer).

Zuerst werden die 10 g Dipyridamol-Wirkstoff mit Aceton zu 100 ml gelöst und anschließend wird diese Mischung mit 6 g Methacrylsäure, 3 g Acrylsäure und 1 g Vinylpyrrolidon sowie mit 2 mg 2,2-Azobisisobutyronitril (AIBN) unter Rühren vermischt (Schritt d) des erfindungsgemäßen Herstellverfahrens für das erfindungsgemäße molekular geprägte Polymer).

Die Mischung wird weiterhin gerührt und unter einer UV Lampe (230 nm) 6 Stunden polymerisiert, wobei innerhalb der erste Stunde der Polymerisierung die Mischung alle 15 Minuten mit einem Vortex (10 Sek.) stark geschüttelt wird (Schritt e) des erfindungsgemäßen Herstellverfahrens für das erfindungsgemäße molekular geprägte Polymer).

Das entstandene Polymer wird als Feststoff nach 6 Stunden zerstoßen und mit 100 ml eines Lösungsmittelsgemischs aus Ethanol/ Methanol (50 : 50 Vol.-%) gewaschen. Anschließend wird das Polymer mit 100 ml Wasser gewaschen.

Die Bindung des erfindungsgemäß molekular geprägten Polymers mit dem Dipyridamol-Wirkstoff kann mittels Hochdruckflüssigkeitschromatographie (high pressure liquid chromatography, HPLC) analytisch über die Konzentrationsabnahme einer physiologischen Stammlösung von Dipyridamol bestimmt werden.

### Ausführungsbeispiel 2: Herstellung eines erfindungsgemäß molekular geprägten Polymers

### Herstellung einer Polymermatrix mit Bindungsselektivität zu Dipyridamol

Zusammensetzung der Polymere 60 % Methacrylsäure, 30 % Acrylsäure, 10 % Vinylpyrrolidon in Aceton alternativ eignen sich THF und Ethanol, wobei in diesen Lösungsmitteln die Wirkstoffkonzentration limitiert ist.
Nach dem Lösen von 10g Wirkstoff (0,5 -10 g) zu 100 ml Aceton werden 6 g Methacrylsäure, 3 g Acrylsäure und 1 g Vinylpyrrolidon zugegeben.
Die Mischung wird gerührt und mit 2 mg AIBN versetz und unter einer UV Lampe (230 nm) polymerisiert. Über die erste Stunde der Polymerisierung wird die Reaktion alle 15 Min. mit einem Vortex (10 Sek.) stark geschüttelt.
Der entstandene Feststoff wird nach 6 Std. entnommen, zerstoßen und mit 100 ml Ethanol/ Methanol (50 :50) gewaschen. Danach wird mit 100 ml Wasser gewaschen.
Die Selektivität auf Dipyridamol wird HPLC-analytisch über die Konzentrationsabnahme einer Stammlösung bestimmt.
Es liegt ein Polymer vor, das Bindungsaffinitäten zum Dipyridamol aufweist.

### Ausführungsbeispiel 3: Herstellung eines erfindungsgemäß beschichteten Implantats

### Beschichtung eines Stentgrundkörpers mit einer Haftvermittlerschicht und einem molekular geprägten Polymer

Der Stentgrundkörper wird unter Feuchtigkeitsausschluss mit Methyl-2-cyanoacrylat besprüht. Der feuchte Stent wird daraufhin in eine Staubkammer überführt, in der sich das pulverisierte und gewaschene Polymer mit molekularer Erkennung auf das Templat (Dipyridamol) befindet. Nach der Abscheidung des molekular geprägten Polymers als Staub auf dem Stent, wird der Stent aus der Staubkammer entnommen und einer Luftfeuchtigkeit von min. 60 % ausgesetzt.

### Ausführungsbeispiel 4: Herstellung eines erfindungsgemäß molekular auf Dipyridamol geprägten Polymers

Es werden 10 g Dipyridamol-Wirkstoff, ca. 100 ml Aceton, 6 g Methacrylsäure, 3 g Methacrylat und 1 g Vinylpyrrolidon und 2 mg 2,2-Azobisisobutyronitril (AIBN) bereitgestellt (Schritte a) bis c) des erfindungsgemäßen Herstellverfahrens für das erfindungsgemäße molekular geprägte Polymer).

Zuerst werden die 10 g Dipyridamol-Wirkstoff mit Aceton zu 100 ml gelöst und anschließend wird diese Mischung mit 6 g Methacrylsäure, 3 g Methacrylat und 1 g Vinylpyrrolidon sowie mit 2 mg 2,2-Azobisisobutyronitril (AIBN) unter Rühren vermischt (Schritt d) des erfindungsgemäßen Herstellverfahrens für das erfindungsgemäße molekular geprägte Polymer).

Die Mischung wird weiterhin gerührt und unter einer UV Lampe (230 nm) 6 Stunden polymerisiert, wobei innerhalb der erste Stunde der Polymerisierung die Mischung alle 15 Minuten mit einem Vortex (10 Sek.) stark geschüttelt wird (Schritt e) des erfindungsgemäßen Herstellverfahrens für das erfindungsgemäße molekular geprägte Polymer).

Das entstandene Polymer wird als Feststoff nach 6 Stunden zerstoßen und mit 100 ml eines Lösungsmittelsgemischs aus Ethanol/ Methanol (50 : 50 Vol.-%) gewaschen. Anschließend wird das Polymer mit 100 ml Wasser gewaschen. Nach dem Trocknen erhält man ein pulverisiertes, molekular geprägtes Polymer mit Bindungsselektivität zu Dipyridamol.

Die Bindung des erfindungsgemäß molekular geprägten Polymers mit dem Dipyridamol-Wirkstoff kann mittels Hochdruckflüssigkeitschromatographie (high pressure liquid chromatography, HPLC) analytisch über die Konzentrationsabnahme einer physiologischen Stammlösung von Dipyridamol bestimmt werden.

### Ausführungsbeispiel 5: Herstellung eines erfindungsgemäß molekular auf Dipyridamol geprägten Polymers

Es werden 10 g Dipyridamol-Wirkstoff, ca. 100 ml Aceton, 6 g Methacrylsäure, 3 g Ethylacrylat und 1 g Vinylpyrrolidon und 2 mg 2,2-Azobisisobutyronitril (AIBN) bereitgestellt (Schritte a) bis c) des erfindungsgemäßen Herstellverfahrens für das erfindungsgemäße molekular geprägte Polymer).

Zuerst werden die 10 g Dipyridamol-Wirkstoff mit Aceton zu 100 ml gelöst und anschließend wird diese Mischung mit 6 g Methacrylsäure, 3 g Ethylacrylat und 1 g Vinylpyrrolidon sowie mit 2 mg 2,2-Azobisisobutyronitril (AIBN) unter Rühren vermischt (Schritt d) des erfindungsgemäßen Herstellverfahrens für das erfindungsgemäße molekular geprägte Polymer).

Die Mischung wird weiterhin gerührt und unter einer UV Lampe (230 nm) 6 Stunden polymerisiert, wobei innerhalb der erste Stunde der Polymerisierung die Mischung alle 15 Minuten mit einem Vortex (10 Sek.) stark geschüttelt wird (Schritt e) des erfindungsgemäßen Herstellverfahrens für das erfindungsgemäße molekular geprägte Polymer).

Das entstandene Polymer wird als Feststoff nach 6 Stunden zerstoßen und mit 100 ml eines Lösungsmittelsgemischs aus Ethanol/ Methanol (50 : 50 Vol.-%) gewaschen. Anschließend wird das Polymer mit 100 ml Wasser gewaschen. Nach dem Trocknen erhält man ein pulverisiertes, molekular geprägtes Polymer mit Bindungsselektivität zu Dipyridamol.

Die Bindung des erfindungsgemäß molekular geprägten Polymers mit dem Dipyridamol-Wirkstoff kann mittels Hochdruckflüssigkeitschromatographie (high pressure liquid chromatography, HPLC) analytisch über die Konzentrationsabnahme einer physiologischen Stammlösung von Dipyridamol bestimmt werden.

Die Reaktion ist im Folgenden gezeigt:

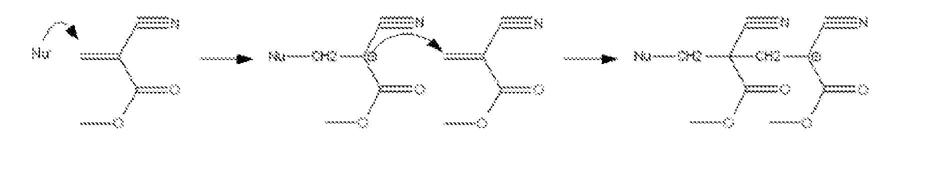

Wasser wirkt hier als Nucleophil und startet die anionische Polymerisation. Der Polymerstab wird so an die Implantatoberfläche geklebt.

## Patentansprüche

1. Implantat zur Implantation in einen menschlichen oder tierischen Organismus mit einer Polymerbeschichtung, **dadurch gekennzeichnet, dass** die Polymerbeschichtung molekular geprägt ist und die molekulare Prägung geeignet ist unter physiologischen Bedingungen in einem menschlichen oder tierischen Organismus
i) ein oder mehrere physiologische Verbindungen eines menschlichen oder tierischen Organismus und/oder
ii) ein oder mehrere systemisch verabreichte Wirkstoffe zu binden.

2. Implantat gemäß Anspruch 1, **dadurch gekennzeichnet, dass** das Polymer ausgewählt wird aus der Gruppe bestehend aus Copolymerisaten von ein oder mehreren Acrylaten und/oder Methacrylaten mit Acrylacetat, Acrylamin, Acrylsäuren, Vinylpyrrolidon, Styrol, Vinylpyridin oder Vinylsaccharid-Copolymerisaten.

3. Implantat gemäß Anspruch 1 oder 2, **dadurch gekennzeichnet, dass**
i) der oder die physiologischen Verbindungen ausgewählt werden aus der Gruppe bestehend aus Wachstumsfaktoren, bevorzugt Transforming Growth Factor, Fibroblast Growth Factor, Angiopoietin 2 und/oder Vascular Endothelial Growth Factor, Enzymen zur Modifikation oder Reduktion extrazellulärer Matrix, bevorzugt Matrix-Metalloproteinasen und dabei besonders bevorzugt Matrix-Metalloproteinase 1, Enzymen zur Fibrinlösung wie, aber nicht ausschließlich, dem Plasmin, bevorzugt aber seinen Aktivatoren oder in Kombination mit diesen, dabei bevorzugt Gewebsplasminogenaktivator und/oder Urokinase Plasminogenaktivator, Enzymen zur Aktivierung von systemisch verabreichten Prodrugs, zum Beispiel Esterasen, Enzyme aus der Reihe der Hydrolasen insbesondere der Lipasen und Proteasen, Enzyme aus der Reihe der Isomerasen, Rezeptoren für Wachstumsfaktoren, bevorzugt aber nicht beschränkt auf Rezeptoren für den Platelet Derived Growth Factor.

4. Implantat gemäß Anspruch 1 oder 2, **dadurch gekennzeichnet, dass**
ii) der oder die Wirkstoffe ausgewählt werden aus der Gruppe bestehend aus Zellproliferationshemmern, Zytostatika, Immunsuppressiva, Statinen, antithrombogen und/oder antikoagulierend wirkenden Wirkstoffen, Vasodilatatoren wie Dipyridamol oder Calciumantagonisten, Kaliumkanalöffnern, Stickstoffmonoxiddonatoren, ACE-Hemmern, Angiotensin-II-Rezeptor-Subtyp-1-Antagonisten, Endothelin-Rezeptorantagonisten und/oder Wirkstoffen, die einer pH-Wert-Verschiebung in den basischen Bereich entgegenwirken können.

5. Implantat gemäß einem der Ansprüche 1 bis 4, **dadurch gekennzeichnet, dass** das Implantat ausgewählt wird aus der Gruppe bestehend aus Herzschrittmacher; Hirnschrittmacher; Herzimplantate; Schrittmacherelektroden; Defibrillationselektroden; Cochleaimplantate; Implantate für die Zahnmedizin; Endoprothesen; Depotimplantate, die dazu dienen, ein Depot eines Wirkstoffs zu bilden; degradierbare oder dauerhafte, koronare oder periphere Stents; degradierbare oder dauerhafte Stents für andere Hohlräume; und local drug delivery (LDD)-Implantate.

6. Verfahren zur Herstellung eines polymerbeschichteten Implantats gemäß einem der Ansprüche 1 bis 5, **dadurch gekennzeichnet, dass** das molekular geprägte Polymer an der Oberfläche des Implantates befestigt ist und dadurch, dass das molekular geprägte Polymer in Kontakt mit Körperflüssigkeiten kommt.

7. Molekular geprägtes Polymer geeignet als Beschichtung für ein polymerbeschichtetes Implantat gemäß einem der Ansprüche 1 bis 5, **dadurch gekennzeichnet, dass** die molekulare Prägung geeignet ist unter physiologischen Bedingungen in einem menschlichen oder tierischen Organismus
i) ein oder mehrere physiologische Verbindungen eines menschlichen oder tierischen Organismus und/oder
ii) ein oder mehrere systemisch verabreichte Wirkstoffe zu binden.

8. Verfahren zur Herstellung eines molekular geprägten Polymers gemäß Anspruch 7, **dadurch gekennzeichnet, dass** das Verfahren folgende Schritte umfasst:
f) Bereitstellung von
i) ein oder mehreren physiologischen Verbindungen eines menschlichen oder tierischen Organismus und/oder
ii) ein oder mehreren systemisch verabreichbaren Wirkstoffen,
g) Bereitstellung von ein oder mehreren polymerisierbare Materialien,
h) Bereitstellung eines oder mehrerer Polymerisationsinitiatoren,
i) Vermischen i) der Verbindung oder der Verbindungen oder ii) des oder der Wirkstoffe aus Schritt a) mit den polymerisierbaren Materialien aus Schritt b) sowie dem oder den Polymerisationsinitiatoren aus Schritt c),
j) Polymerisieren der Mischung aus Schritt d) und
k) Entfernen i) des oder der Verbindungen oder ii) des oder der Wirkstoffe zumindest aus der Oberfläche des Polymers durch Waschung des Polymers aus Schritt e) mit einem oder mehreren geeigneten Lösungsmittel und/oder Lösungsmittelgemisch.

9. Verfahren gemäß Anspruch 8, **dadurch gekennzeichnet, dass** der Polymerisationsinitiator aus Schritt c) 2,2-Azobisisobutyronitril (AIBN) ist.

10. Verfahren gemäß Anspruch 8 oder 9, **dadurch gekennzeichnet, dass** in Schritt d) die Verbindung oder die Verbindungen aus Schritt a) zuerst in einem Lösungsmittel oder Lösungsmittelgemisch gelöst werden, ausgewählt aus der Gruppe bestehend aus Aceton, Dioxan, Wasser, Chloroform, Tetrahydrofuran und/oder Wasser.

11. Verfahren gemäß einem der Ansprüche 8 bis 10, **dadurch gekennzeichnet, dass** in Schritt e) mittel UV-Strahlung polymerisiert wird.

12. Verfahren gemäß einem der Ansprüche 8 bis 11, **dadurch gekennzeichnet, dass** in Schritt f) das oder die geeigneten Lösungsmittel und/oder Lösungsmittelgemische ausgewählt werden aus der Gruppe bestehend aus Methanol, Ethanol und/oder Wasser.

13. Verwendung eines molekular geprägten Polymers gemäß Anspruch 7 als Beschichtung für ein polymerbeschichtetes Implantat gemäß einem der Ansprüche 1 bis 5 für einen menschlichen oder tierischen Organismus.

14. Verwendung eines molekular geprägten Polymers gemäß Anspruch 7 zur Herstellung eines polymerbeschichteten Implantats gemäß einem der Ansprüche 1 bis 5.

15. Verfahren zur Behandlung oder Prophylaxe einer Stenose, zur Behandlung von Erkrankungen der Gefäße und/oder der Erkrankungen von durch diese Gefäße versorgten Geweben, einschließlich Tumoren, **dadurch gekennzeichnet, dass** ein Implantat gemäß einem der Ansprüche 1 bis 5 in einen menschlichen oder tierischen Organismus implantiert wird.
